# EUROPEAN PATENT APPLICATION

(11) **EP 4 548 779 A1**
(43) Date of publication of application: **07.05.2025**
(21) Application number: 23831483.5
(22) Date of filing: 28.06.2023
(51) Int. Cl.: A23L 33/10, A23C 9/152, A23L 2/38, A23L 2/52, A23L 33/125, A61K 31/7008, A61K 31/702, A61K 31/715, A61K 47/54, A61K 47/64, A61P 11/00

(54) **NUTRITIONAL COMPOSITION FOR PREVENTING BRONCHITIS**

(30) Priority: 29.06.2022 JP 2022104518
(71) Applicant: Megmilk Snow Brand Co. Ltd., Sapporo-shi, Hokkaido 065-0043 (JP)
(72) Inventor: YAMAGUCHI, Toshiyuki, Sapporo-shi, Hokkaido 065-0043 (JP); HIGUCHI, Junichi, Sapporo-shi, Hokkaido 065-0043 (JP); FUKUDOME, Hirofumi, Sapporo-shi, Hokkaido 065-0043 (JP); SAKAI, Fumihiko, Sapporo-shi, Hokkaido 065-0043 (JP); TSUJIMORI, Yuta, Sapporo-shi, Hokkaido 065-0043 (JP); TAKAHASHI, Tomoki, Sapporo-shi, Hokkaido 065-0043 (JP)
(74) Representative: Mathys & Squire
(86) International application number: PCT/JP2023/023898
(87) International publication number: WO 2024/005043

(57) **Abstract**

A problem to be solved by the present invention is to find a component for preventing the onset of bronchitis from breast milk, to provide a novel nutritional composition. The present invention relates to a nutritional composition for preventing the onset of bronchitis wherein N-acetylneuraminic acid, 6'-sialyllactose, α-2,6-disialo N-linked glycan, or a disialo N-linked glycan with a specific structure are contained as active ingredients. This nutritional composition is an effective composition not only for infants but also for elderly people and people with underlying diseases who are at high risk of contracting the bronchitis, so the composition is efficacious as the nutritional composition for preventing the onset of bronchitis when it is added to foods and nutritional compositions for adults and the elderly.

## Description

### TECHNICAL FIELD

The present invention relates to a nutritional composition for preventing bronchitis. More particularly, the present invention relates to a nutritional composition for preventing bronchitis, which contains N-acetylneuraminic acid as an active ingredient.

### BACKGROUND ART

Bronchitis is a disease causing inflammation in which viruses or bacteria infect the airways where the trachea branches (bronchi). Many of the causes of bronchitis are viral infections such as a respiratory syncytial (RS) virus, a rhinovirus, an adenovirus, a coronavirus, and an influenza virus. Bacterial pathogens such as a mycoplasma and a Bordetella pertussis can also cause the bronchitis. After an initial infection with these pathogens, bacteria such as a Streptococcus pneumococcus and a Haemophilus influenzae can cause a secondary infection, leading to severe diseases.

Since immune functions have not yet matured in infancy, infants are at a high risk of contracting the bronchitis. In particular, infants up to about 3 months old have thin bronchi, so that their ability to expel phlegm is so weak as to be at a high risk of becoming severe due to a spread of inflammation.

Furthermore, elderly people whose immune functions are declining are also at a high risk of contracting the bronchitis. The bronchitis can progress to pneumonia if it worsens, leading to serious, life-threatening symptoms.

Therefore, preventing the bronchitis is important for everyone, from infants to elderly people, to lead a healthy social life. So far, various studies have been conducted on methods of treating and preventing the bronchitis. Regarding treatment methods after contracting the bronchitis, administration of anti-influenza virus drugs, treatment with various antibiotics, treatment with cough suppressants and expectorants, etc., have been developed. Patent Document 1 discloses an antiulcer agent or bifidobacterium growth promoter containing a specific sialic acid derivative as an active ingredient. The sialic acid derivative is also proved to have therapeutic effects on infectious diseases caused by viruses such as the influenza virus, a rotavirus, and a herpes virus. But the document does not disclose a preventive effect of the sialic acid derivative on the bronchitis. The patent document discloses the preventive effect of the sialic acid derivative on diarrhea caused by the rotavirus. However, the rotavirus is not a causative virus of the bronchitis, so that the preventive effect of the sialic acid derivative on the bronchitis is not disclosed. Thus, there is no effective method for preventing the bronchitis, and the only thing to be done is a general encouragement on handwashing and gargling.

Incidentally, a breast milk is known to contain various components which are able to reduce the risk of the infectious diseases (Patent Document 2). In particular, carbohydrates represented by oligosaccharides contained in the breast milk is reported to inhibit a stage at which certain viruses attach to host cells (Patent Document 3).

For searching for the active ingredients preventing the onset of bronchitis, the present inventors focused on ingredients contained in the breast milk. However, it has been specifically unclear whether these are able to prevent the onset of bronchitis itself, and what components in the breast milk can prevent the onset of bronchitis could not be inferred.

### PATENT LITERATURE

Patent Document 1: JP 3,930,559 B
Patent Document 2: JP 2,514,375 B
Patent Document 3: JP 3,789,146 B

### SUMMARY OF INVENTION

### TECHNICAL PROBLEM

The present inventors conceived that if a component able to prevent the onset of bronchitis could be found among breast milk components, it might become possible to supply a nutritional composition for infants preventing the onset of bronchitis by incorporating the component into the nutritional composition for infants. The component is an effective component not only for infants but also for elderly people and people with underlying diseases who are at a high risk of bronchitis, so that the inventors thought that it will be possible to incorporate the component into foods and nutritional compositions for adults and the elderly to provide it as the nutritional composition for preventing the onset of bronchitis.

The present invention addresses a problem of finding a component preventing the onset of bronchitis from the breast milk components to provide a new nutritional composition.

### SOLUTION TO PROBLEM

For searching for components in a breast milk preventing the onset of bronchitis, the present inventors statistically analyzed a relationship between amounts of carbohydrate components in the breast milk and bronchitis medical histories on infants. As a consequence, the inventors found that N-acetylneuraminic acid, 6'-sialyllactose, α-2,6-disialo N-linked glycan, and a disialo N-linked glycan with a specific structure in the breast milk have a preventive effect on the bronchitis, completing the present invention.

That is, the present invention relates to a nutritional composition for preventing the onset of bronchitis, including one or more active ingredients selected from a group consisting of N-acetylneuraminic acid, 6'-sialyllactose, α-2,6-disialo N-linked glycan, and disialo N-linked glycan with a specific structure. Specifically, the present invention has the following configurations.

<1> A nutritional composition for preventing bronchitis containing N-acetylneuraminic acid as an active ingredient.
<2> The nutritional composition according to <1>, wherein N-acetylneuraminic acid is bound to any one or more of a protein, a peptide, an oligosaccharide, or a lipid.
<3> The nutritional composition according to <1> or <2>, wherein N-acetylneuraminic acid is an α-2,6-linked N-acetylneuraminic acid.
<4> The nutritional composition according to <3>, wherein N-acetylneuraminic acid exists as 6'-sialyllactose.
<5> The nutritional composition according to <3>, wherein N-acetylneuraminic acid exists as an N-linked glycan.
<6> The nutritional composition according to <5>, wherein the N-linked glycan is α-2,6-disialo N-linked glycan.
<7> The nutritional composition according to <6>, wherein α-2,6-disialo N-linked glycan has a structure shown in (1) or (2) below.
   (1) Neu5Acα2-6Galβ1-4GlcNAcβ1-2Manα1-3(Neu5Acα2-6Galβ1-4GlcNAcβ1-2Manα1-6)Manβ1-4GlcNAcβ1-4GlcNAc
   (2) Neu5Acα2-6Galβ1-4GlcNAcβ1-2Manα1-3(Neu5Acα2-6Galβ1-4GlcNAcβ1-2Manα1-6)Manβ1-4GlcNAcβ1-4(Fucα1-6)GlcNAc
<8> The nutritional composition according to any one of <1> to <7>, wherein it is for infants.
<9> A food and drink for preventing bronchitis comprising the nutritional composition for preventing bronchitis according to any one of <1> to <8>.
<10> A pharmaceutical for preventing bronchitis comprising the nutritional composition for preventing bronchitis according to any one of <1> to <8>.

The present invention also has configurations which follow.
<11> A method for preventing bronchitis, comprising a step of administering to a human a nutritional composition containing an effective amount of N-acetylneuraminic acid.
<12> The method for preventing bronchitis according to <11>, wherein N-acetylneuraminic acid is bound to any one or more of a protein, a peptide, an oligosaccharide, or a lipid.
<13> The method for preventing bronchitis according to <11> or <12>, wherein N-acetylneuraminic acid is an α-2,6-linked N-acetylneuraminic acid.
<14> The method for preventing bronchitis according to <13>, wherein N-acetylneuraminic acid is present as 6'-sialyllactose.
<15> The method for preventing bronchitis according to <13>, wherein N-acetylneuraminic acid is an N-linked glycan.
<16> The method for preventing bronchitis according to <15>, wherein the N-linked glycan is α-2,6-disialo N-linked glycan.
<17> The method for preventing bronchitis according to <16>, wherein α-2,6-disialo N-linked glycan has a structure shown in (1) or (2) below.
   (1) Neu5Acα2-6Galβ1-4GlcNAcβ1-2Manα1-3 (Neu5Acα2-6Galβ1-4GlcNAcβ1-2Mana1-6) Manβ1-4GlcNAcβ1-4GlcNAc
   (2) Neu5Acα2-6Galβ1-4GlcNAcβ1-2Manα1-3 (Neu5Acα2-6Galβ1-4GlcNAcβ1-2Manα1-6) Manβ1-4GlcNAcβ1-4 (Fucα1-6) GlcNAc
<18> The method for preventing bronchitis according to any one of <11> to <17>, wherein the human is an infant.
<19> The method for preventing bronchitis according to any one of <11> to <18>, wherein the nutritional composition is a food or a drink.
<20> The method for preventing bronchitis according to any one of <11> to <18>, wherein the nutritional composition is a pharmaceutical.

### ADVANTAGEOUS EFFECTS OF INVENTION

According to the present invention, the onset of bronchitis can be prevented by a nutritional composition containing N-acetylneuraminic acid as an active ingredient. The ingredient can be an effective ingredient not only for infants but also for elderly people and those with underlying diseases that are at a high risk of bronchitis. Thus, the present invention enables the prevention of the onset of bronchitis in adults and the elderly.

### BRIEF DESCRIPTION OF DRAWINGS

[Fig. 1] Fig. 1 shows effects of N-acetylneuraminic acid concentrations in a breast milk on the onset of bronchitis in infants.
[Fig. 2] Fig. 2 shows effects of 6'-SL concentrations in the breast milk on the onset of bronchitis in infants.
[Fig. 3] Fig. 3 shows effects of disialo N-linked glycan A concentrations in the breast milk on the onset of bronchitis in infants.
[Fig. 4] Fig. 4 shows effects of disialo N-linked glycan B concentrations in the breast milk on the onset of bronchitis in infants.
[Fig. 5] Fig. 5 shows effects of α-2,6-disialo N-linked glycan concentrations in the breast milk on the onset of bronchitis in infants.

### DESCRIPTION OF EMBODIMENTS

### (N-Acetylneuraminic Acid)

N-acetylneuraminic acid of the present invention may be in a free state or in a state bound to other sugars as an oligosaccharide. N-acetylneuraminic acid may be in the state bound to a peptide and/or a protein or a lipid. N-acetylneuraminic acid may be derived from milk, egg, swallow's nest, or a fermentation method of genetically modified bacteria, and may be any of a synthetic, a semi-synthetic, or a natural product. A natural material containing the natural product may be added as it is as a supply source to a nutritional composition of the present invention. As used herein, the natural product means a product extracted from a natural material containing N-acetylneuraminic acid by well-known methods, a product made by crudely purifying the extract, or a product made by further highly purifying them. For example, glycomacropeptides derived from milk and a sialylglycopeptide made by condensing glycan portions of the glycomacropeptides are included.

The linkage mode of N-acetylneuraminic acid is desirably an α-2,6 bond in the invention. A-2,6-linked N-acetylneuraminic acid refers to N-acetylneuraminic acid bound, via the α-2,6 bond, to a galactose residue in oligosaccharide or glycoprotein glycan, or to N-acetylgalactosamine residue. More specifically, N-acetylneuraminic acid refers to a compound having a Neu5Acα2-6Gal structure or a Neu5Acα2-6GalNAc structure.

### (6'-Sialyllactose)

In the present invention, a glycan containing N-acetylneuraminic acid is desirably present as 6'-sialyllactose (Neu5Acα2-6Galβ1-4Glc). 6'-sialyllactose (hereinafter sometimes simply referred to as 6'-SL) may be derived from milk or the fermentation method of genetically modified bacteria, and may be any of the synthetic, semi-synthetic, or natural product. The natural material containing the natural product may be added as it is as the supply source to the nutritional composition of the invention. As used herein, the natural product means a product extracted from the natural material containing 6'-sialyllactose by the well-known methods, a product made by crudely purifying the extract, or a product made by further highly purifying them.

### (N-Linked Glycan / N-Linked Sugar Chain)

In the invention, the glycan containing N-acetylneuraminic acid is desirably present as N-linked glycan (N-linked sugar chain). The N-linked glycan comprising N-acetylneuraminic acid of the invention may be in the free state or in the state bound to the peptide and/or the protein. It may be derived from milk, egg, meat, microbes, or the fermentation method of genetically modified bacteria, and may be any of the synthetic, semi-synthetic, or natural product. The natural material containing the natural product may be added intactly as the supply source to the nutritional composition of the invention. As used herein, the natural product means the product extracted from the natural material containing N-linked glycan by the well-known methods, the product made by crudely purifying that, or the product made by further highly purifying them.

### (α-2,6-Disialo N-Linked Glycan)

α-2,6-disialo N-linked glycan in the invention refers to the N-linked glycan reducing the onset of bronchitis. A-2,6-disialo N-linked glycan of the invention may be in the free state or in the state bound to the peptide and/or the protein. They may be derived from milk, egg, meat or the fermentation method of genetically modified bacteria, and may be any of the synthetic, semi-synthetic, or natural product. The natural material containing the natural product may be added as it is as the supply source to the nutritional composition of the invention. Here, the natural product means the product extracted from the natural material containing α-2,6-Disialo N-Linked Glycan by the well-known methods, the products made by crudely purifying that, or the products made by further highly purifying them.

Specific examples of α-2,6-disialo N-linked glycans in the present invention include Neu5Acα2-6Galβ1-4GlcNAcβ1-2Manα1-3 (Neu5Aca2-6Galβ1-4GlcNAcβ1-2Manα1-6) Manβ1-4GlcNAcβ1-4GlcNAc (hereinafter, sometimes simply referred to as a disialo N-linked glycan A) and Neu5Acα2-6Galβ1-4GlcNAcβ1-2Manα1-3 (Neu5Acα2-6Galβ1-4GlcNAcβ1-2Manα1-6) Manβ1-4GlcNAcβ1-4 (Fucα1-6) GlcNAc (hereinafter, sometimes simply referred to as a disialo N-linked glycan B). In addition, for example, the N-linked glycans with two molecules of Neu5Acα-2,6 bonds at non-reducing terminals are included such as Neu5Acα2-6Galβ1-4 (Fucα1-3) GlcNAcβ1-2Manα1-3 (Neu5Acα2-6Galβ1-4GlcNAcβ1-2Manα1-6) Manβ1-4GlcNAcβ1-4GlcNAc, Neu5Acα2-6Galβ1-4GlcNAcβ1-2Manα1-3 (Neu5Acα2-6Galβ1-4 (Fucα1-3) GlcNAcβ1-2Manα1-6) Manβ1-4GlcNAcβ1-4GlcNAc, and Neu5Acα2-6Galβ1-4GlcNAcβ1-2 (Neu5Acα2-6Galβ1-4GlcNAcβ1-4) Manα1-3 (Galβ1-4GlcNAcβ1-2Manα1-6) Manβ1-4GlcNAcβ1-4GlcNAc.

The nutritional composition of the present invention is required to contain an effective amount of N-acetylneuraminic acid for preventing the bronchitis. A preferable range of a required amount of the nutritional composition of the invention per 100 g of solid matter is showed below.

N-acetylneuraminic acid is preferably 170 mg/100 g solid or more, and more preferably 189 mg/100 g solid or more. In a case that the nutritional composition is a powdered milk, a milk after preparation preferably contains 221 mg/L or more of N-acetylneuraminic acid, and more preferably 246 mg/L or more.

The nutritional composition of the invention preferably contains 46.9 mg/100 g solid or more of 6'-sialyllactose, and more preferably 104 mg/100 g solid or more.

The milk after preparation preferably contains 61.0 mg/L or more of 6'-SL, and more preferably 135 mg/L or more.

The nutritional composition of the invention preferably contains 0.198 µmol/100 g solid or more of α-2,6-disialo N-linked glycan, and more preferably 0.338 µmol/100 g solid or more. The milk after preparation preferably contains 0.257 µmol/L or more of α-2,6-disialo N-linked glycan, and more preferably 0.440 µmol/L or more.

The nutritional composition of the invention preferably contains 0.0728 µmol/100 g solid or more of the disialo N-linked glycan A, and more preferably 0.241 µmol/100 g solid or more. The milk after preparation preferably contains 0.0947 µmol/L or more of the disialo N-linked glycan A, and more preferably 0.313 µmol/L or more.

The nutritional composition of the invention preferably contains 0.0935 µmol/100 g solid or more of the disialo N-linked glycan B, and more preferably 0.188 µmol/100 g solid or more. The milk after preparation preferably contains 0.122 µmol/L or more of the disialo N-linked glycan B, and more preferably 0.245 µmol/L or more.

### (Nutritional Composition, Food and Drink, and Pharmaceutical)

The nutritional composition of the present invention for preventing the bronchitis may be a composition containing the effective amount of N-acetylneuraminic acid and the like. The nutritional composition of the invention can contain the proteins, the lipids, carbohydrates, vitamins, minerals and the like, other than an active ingredient. The nutritional composition of the present invention is used as the nutritional composition for infants, adults, or elderly people.

The nutritional compositions for infants include powder and liquid infant formulas, powder toddler formulas (a follow-up milk and a growing-up milk), powder formulas for low-birth-weight infants, allergen-free foods for milk allergies, lactose-free foods for lactose intolerance and the like.

The nutritional compositions for adults and the elderly include powder and liquid milk formulas for adults, the allergen-free foods for cow milk allergies, the lactose-free foods for lactose intolerance, supplements, and the like.

As foods and drinks of the present invention for preventing the bronchitis which contain N-acetylneuraminic acid and the like, any foods and drinks with the effective amount of N-acetylneuraminic acid are preferable. N-acetylneuraminic acid and the like may be added to raw materials during a manufacturing process of the foods and drinks. The examples of the foods and drinks include, but are not limited to, a dairy product such as a cheese, a fermented milk, a lactic acid bacteria drink, a butter, a margarine, and the like, a beverage such as a milk drink, a fruit juice drink, and a soft drink, a jelly, a candy, an egg product such as a pudding and a mayonnaise, and a confectionery and bread such as a butter cake.

The nutritional composition of the invention for preventing the bronchitis can be used as it is as the raw material for pharmaceuticals. The pharmaceuticals of the invention may be manufactured by ordinary methods such as tablets, capsules, powders, granules, powdered medicines, and syrups.

The pharmaceuticals of the invention can be formulated mixing N-acetylneuraminic acid and the like and pharmaceutically permitted excipients, stabilizers, flavorings, and the like. The composition containing N-acetylneuraminic acid and the like can be dried as it is to be used as the powder or the powdered medicine. Within a range which does not interfere an effect of preventing the bronchitis, any of the excipient, a binder, a disintegrant, a lubricant, a corrective, a suspending agent, a coating agent, and other optional drugs can be mixed to be formulated.

### EXAMPLES

### [Example 1]

Various carbohydrate components were measured for 153 breast milk samples, analyzing a relationship with a presence or absence of the onset of bronchitis in infants during the first year of life using a logistic regression. The methods and results are shown below.

### 1. Methods

### (1) Investigation of Breast Milk Samples and Infection Histories of infants

Bean Stalk Snow Co., Ltd. Conducted a prospective cohort study (UMIN ID 000015494) from 2014 to 2019 for investigating the relationship between the breast milk components of Japanese and a health status of mothers and children in Japan. In the study, breast milk samples collected from 1,210 mothers and questionnaire results of investigating the health status of mothers and children were used in the study. In addition, 200 samples were randomly selected from the 1,210 samples. The breast milk collected between 1 and 2 months after childbirth was used as the breast milk samples for analysis. Parents filled out questionnaires investigating the health status of their infants six times in total, every two months for one year after birth, and the results were collected. From the questionnaire results, the results were compiled of whether or not the infants were diagnosed with the bronchitis by a doctor within one year after the childbirth. If a description of a presence or absence of the onset of bronchitis was unclear, they were excluded from the study.

As a result, in the questionnaires conducted six times in total in a year, for 153 samples, questionnaires were answered correctly about whether the infants had been diagnosed with the bronchitis, and the samples became subjects to be analyzed in the study. Of the 153 samples, 18 samples were from infants diagnosed with the bronchitis within one year of birth.

### (2) Measurement of Various Carbohydrate Components in Breast Milk

### (2-1) Measurement of Total N-Acetylneuraminic Acid

800 µL of an ultrapure water and 100 µL of a 1N sulfuric acid were added to 100 µL of the breast milk from the 153 samples to be analyzed, and then heated at 80°C for 45 minutes. Cooling that to a room temperature, adding 1 mL of a 0.1N sodium hydroxide aqueous solution, and then an ultrafiltration membrane treatment with a molecular weight cutoff of 10,000 was performed, analyzing a permeate solution by an HPLC. For the HPLC, a DIONEX ICS-5000DP system (Thermo Fisher Scientific Co., Ltd.) connected to an electrochemical detector was used, and a CarboPac PA1 column (Thermo Fisher Scientific Co., Ltd.) was used for a column. As mobile phases, three solutions, which are the ultrapure water (solution A), a 200 mM sodium hydroxide solution (solution B), and a 100 mM sodium hydroxide solution containing 600 mM sodium acetate (solution C), were passed at a flow rate of 1 mL/min. For the first 7 minutes, a 45% solution B and a 10% solution C were passed. Thereafter, the solution B was linearly decreased down to 37.5% by 10 minutes, and the solution C was linearly increased up to 25%. From 10 to 20 minutes, a 37.5% solution B and a 25% solution C were passed. From 20.1 to 25 minutes, the 45% solution B and the 10% solution C were passed. The amount of a total N-acetylneuraminic acid in each breast milk sample was calculated from a calibration curve prepared using a standard preparation of N-acetylneuraminic acid with a known concentration.

### (2-2) Measurement of Oligosaccharides

Five hundred and eighty (580) µL of the ultrapure water was added to 20 µL of the breast milk from the 153 samples to be analyzed. Thereafter, the ultrafiltration membrane treatment with the molecular weight cutoff of 10,000 was performed, and the permeate solution was analyzed by the HPLC. For the HPLC, the DIONEX ICS-5000DP system (Thermo Fisher Scientific Co., Ltd.) connected to the electrochemical detector was used and the CarboPac PA1 column (Thermo Fisher Scientific Co., Ltd.) was used for the column. As the mobile phases, the three solutions, which are the ultrapure water (solution A), the 200 mM sodium hydroxide solution (solution B), and the 100 mM sodium hydroxide solution containing 600 mM sodium acetate (solution C), were passed at the flow rate of 1 mL/min. A 50% solution B was passed for the first 10 minutes. Thereafter, the solution B was linearly decreased down to 47.5% by 18 minutes, and the solution C was linearly increased up to 5%. From 18 to 28 minutes, a 47.5% solution B and a 5% solution C were passed. From 28 to 32 minutes, the solution B was linearly decreased down to 42%, and the solution C was linearly increased up to 16%. From 32 to 39 minutes, a 42% solution B and a 16% solution C were passed. Thereafter, from 39 to 43 minutes, a 30% solution B and a 40% solution C were passed. Each oligosaccharide content in each breast milk sample was calculated from the calibration curve prepared using the standard preparation with known concentrations of a 2'-fucosyllactose, a 3-fucosyllactose, a 3'-sialyllactose (hereinafter sometimes simply referred to as a 3'-SL), a 6'-sialyllactose (hereinafter sometimes simply referred to as 6'-SL), a lactodifucotetraose, a lacto-N-tetraose, a lacto-N-neotetraose, a lacto-N-fucopentaose I, a lacto-N-fucopentaose II, a lacto-N-difucohexaose I, and a lacto-N-difucohexaose II. The total value of these 11 types of oligosaccharides was defined as "total oligosaccharides".

### (2-3) Measurement of N-Linked Glycans

Ten (10) µL of a 3M sodium borohydride aqueous solution was added to 20 µL of the breast milk from the 153 samples to be analyzed, leaving them to stand at the room temperature for 30 minutes. Then, 7.5 µL of an acetic acid was added, leaving them to stand at the room temperature for 30 minutes. 15 µL of a 1M ammonium bicarbonate aqueous solution and 7.5 µL of the ultrapure water were added. Next, 5 µL of a 400 mM dithiothreitol aqueous solution containing a 5% sodium lauryl sulfate was added to be heated at 100°C for 10 minutes, denaturing proteins. Cooling them to the room temperature, and 10 µL of a 123 mM iodoacetamide aqueous solution was added, leaving them to stand at the room temperature for 1 hour. 7 µL of a 500 mM sodium acetate aqueous solution (pH 6.0) and 9 µL of a 10% NP-40 solution were added. Thereafter, 10 µL of a 500 units/µL Peptide-N-glycanase F solution was added. Then, the mixture was left to stand at 37°C for 24 hours, releasing N-linked glycans. Adding 109 µL of the ultrapure water, and then the entire amount was introduced into a Vivaspin 500 (Sartorius Co., Ltd.) with the molecular weight cutoff of 10,000. The mixture was centrifuged at 15,000×g and 25° C, for 10 minutes, retrieving the permeate solution. 50 µL of an acetonitrile was added to 50 µL of the permeate solution to be subjected to an LC/MS analysis. The LC/MS analysis was performed as follows. For the HPLC, an UltiMate 3000 (Thermo Fisher Scientific Co., Ltd.) was used, and a Q-Exactive (Thermo Fisher Scientific Co., Ltd.) was used for an MS. For the column, a GlycanPac AXH-1 (Thermo Fisher Scientific Co., Ltd.) was used, and a column temperature was set to 40°C. As the mobile phases, the acetonitrile (solution A) and a 50 mM ammonium formate aqueous solution (solution B) with the pH adjusted to 4.4 were used to be passed at the flow rate of 0.4 mL/min. After an introduction of samples, a proportion of the solution B was increased linearly from 10% to 35% by 55 minutes. Thereafter, a 35% solution B was passed from 55 minutes to 65 minutes. A spray voltage of an ESI probe was 3.5 kV, a capillary temperature was 275°C, and a nitrogen gas was used for a sheath gas, an auxiliary gas, and a collision gas. An S-Lens RF Level was set to 90. MS spectra were acquired by a full scan measurement in the range of m/z 400-2000 in positive mode. The MS spectra acquired were analyzed using a Compound Discover 2.1 (Thermo Fisher Scientific Co., Ltd.). They were compared with a glycan database constructed from a previously reported N-linked glycans of milk proteins, to detect glycans. The Compound Discover created an extraction chromatogram corresponding to a molecular ion peak of each glycan, calculating a signal area value of each glycan detected in each breast milk sample. The calculated signal area value of the glycans was used as a relative quantitative value of each glycan. The N-linked glycans detected were sixteen types. The sum of their area values was used as a relative value of "total N-linked glycans".

### (2-4) Measurement of O-Linked Glycans

A glycan release reagent, which is included in an O-linked glycan analysis kit EZGlyco Prep kit (Sumitomo Bakelite Co., Ltd.), was added to the 153 breast milk samples to be analyzed, heating them at 50°C for 20 minutes. After a dilution with the ultrapure water, they were introduced into the Vivaspin 500 with the molecular weight cutoff of 10,000. They were centrifuged at 15,000×g and 25°C for 15 minutes, to collect the permeate solution. 50 µL of the acetonitrile was added to 50 µL of the permeate solution, which was subjected to the LC/MS analysis. The UltiMate 3000 was used for the HPLC, and the Q-Exactive was used for the MS. The GlycanPac AXH-1 column was used for the column, and the column temperature was set to 40°C. As the mobile phases, the acetonitrile (solution A) and the 50 mM ammonium formate aqueous solution (solution B) with the pH adjusted to 4.4 were used to be passed at the flow rate of 0.4 mL/min. After the introduction of samples, the proportion of the solution B was increased linearly from 10% to 35% by 55 min. Thereafter, from 55 min to 65 min, the 35% solution B was passed. The spray voltage of the ESI probe was 3.5 kV, the capillary temperature was 275°C, and the nitrogen gas was used for the sheath gas, the auxiliary gas, and the collision gas. The S-Lens RF Level was set to 90. The MS spectra were acquired by the full scan measurement in the range of m/z 300-2000 in positive mode. The MS spectra acquired were analyzed using the Compound Discover 2.1 (Thermo Fisher Scientific Co., Ltd.). They were compared with the O-linked glycan database, to detect the glycans. The Compound Discover created the extraction chromatogram corresponding to the molecular ion peak of each glycan, calculating the signal area value of each glycan detected in each breast milk sample. The calculated signal area value of the glycans was used as the relative quantitative value of each glycan. The O-linked glycans detected were twelve types. The sum of their area values was used as the relative value of "total O-linked glycans".

### (3) Determination of Structures of Disialo N-Linked Glycans A and B

### (3-1) Structural Analysis

An LC/MS/MS measurement was performed on one of the 153 samples prepared in the above "Measurement of N-Linked glycans", performing a structural analysis of disialo N-linked glycans A and B. The LC/MS/MS analysis was performed as follows. The UltiMate 3000 was used for the HPLC and the Q-Exactive MS was used for the MS. The GlycanPac AXH-1 column was used for the column, and the column temperature was set to 40°C. As the mobile phases, the acetonitrile (solution A) and the 50 mM ammonium formate aqueous solution (solution B) with the pH adjusted to 4.4 were used to be passed at the flow rate of 0.4 mL/min. After the introduction of samples, the proportion of the solution B was increased linearly from 10% to 35% by 55 min. Thereafter, from 55 min to 65 min, the 35% solution B was passed. The spray voltage of the ESI probe was 3.5 kV, the capillary temperature was 275°C, and the nitrogen gas was used for the sheath gas, the auxiliary gas, and the collision gas. The S-Lens RF Level was set to 90. MS/MS spectra were acquired in positive mode and data-dependent Top10 mode. The MS/MS spectra acquired were analyzed using SimGlycan Software (PREMIER Biosoft International), analyzing the structures of the disialo N-linked glycans A and B.

### (3-2) Linkege Mode Analysis

For determining linkage modes of N-acetylneuraminic acids in the disialo N-linked glycans A and B, the LC/MS analysis was performed after a process using a sialidase. Either an sialidase specifically degrading α-2,3 bonds (α-2,3-Sialidase) or a sialidase degrading both α-2,3 and α-2,6 bonds were used as the sialidase. In one sample identical to that in (3-1) above, 37 µL of the ultrapure water and 5 µL of the buffer attached to the enzyme were added to 5 µL of N-linked glycan solution. 3 µL of an α-2,3-Sialidase or Neuraminidase diluted 10-fold with the buffer attached to the enzyme was added to be incubated at 37°C for 15 minutes. After heating them in a boiling water for 5 minutes for inactivating the enzyme, 50 µL of the acetonitrile was added to be subjected to the LC/MS analysis. The LC/MS analysis was performed as follows. The UltiMate 3000 was used for the HPLC, and the Q-Exactive was used for the MS. The GlycanPac AXH-1 was used for the column, and the column temperature was set to 40°C. As the mobile phases, the acetonitrile (solution A) and the 50 mM ammonium formate aqueous solution (liquid B) with the pH adjusted to 4.4 were used to be passed at the flow rate of 0.4 mL/min. After the introduction of samples, the proportion of the solution B was increased linearly from 10% to 35% by 55 minutes. Thereafter, from 55 minutes to 65 minutes, the 35% solution B was passed. The spray voltage of the ESI probe was 3.5 kV, the capillary temperature was 275°C, and the nitrogen gas was used for the sheath gas, the auxiliary gas, and the collision gas. The S-Lens RF Level was set to 90. The acquired MS spectra were analyzed using an Xcalibur software (Thermo Fisher Scientific Co., Ltd.).

### (4) Absolute Quantification of Disialo N-Linked Glycans A and B

Absolute amounts of the disialo N-linked glycans A and B in the breast milk which have been found to have relevance to the onset of bronchitis were measured. The content of the disialo N-linked glycan A in the breast milk was measured by a standard addition method using a standard substance of a disialo N-linked glycopeptide. The absolute amount of the disialo N-linked glycan B in the breast milk was calculated by multiplying the absolute amount of the disialo N-linked glycan A by a relative ratio of the area value of the disialo N-linked glycan B to that of the disialo N-linked glycan A obtained from LC/MS results.

A method for measuring the disialo N-linked glycan A by the standard addition method is described below. Ten (10) µL of the 3 M sodium borohydride aqueous solution was added to 20 µL of the breast milk of one sample randomly selected from the 153 breast milk samples to be analyzed, leaving them to stand at the room temperature for 30 minutes. Then, 7.5 µL of the acetic acid was added, leaving them to stand at the room temperature for 30 minutes. Fifteen (15) µL of the 1 M ammonium bicarbonate aqueous solution was added, then 7.5, 5.5, 3.5, or 1.5 µL of the ultrapure water is added. Moreover, 0, 2, 4, or 6 µL of an aqueous solution, in which a sialyl glycopeptide (Tokyo Chemical Industry Co., Ltd.) is prepared to be 20 µg/mL for use as the standard substance for a glycopeptide bound to the disialo N-linked glycan A, was added. Following that, 5 µL of a 120 mM dithiothreitol aqueous solution was added to be heated at 60°C for 30 minutes. Then, 10 µL of the 123 mM iodoacetamide aqueous solution was added to be left to stand at the room temperature for 1 hour. 20 µL of a 40 units/µL trypsin solution was added to be incubated at 37°C for 1 hour. The mixture was heated at 100°C for 5 minutes, to inactivate trypsin. Then, the 500 units/µL Peptide-N-glycanase F solution was added to be left to stand at 37°C for 18 hours, releasing the N-linked glycans. Using N-linked glycan preparation kit BlotGlyco (Sumitomo Bakelite), the released N-linked glycans were labeled with a 2-aminobenzamide and purified to be subjected to the LC/MS analysis. The UltiMate 3000 is used for the HPLC, and the Q-Exactive is used for the MS. The GlycanPac AXH-1 was used for the column, and the column temperature was set at 40°C. As the mobile phases, the acetonitrile (solution A), and the 50 mM ammonium formate aqueous solution (solution B) with the pH adjusted to 4.4, were used to be passed at the flow rate of 0.4 mL/min. After the introduction of samples, the proportion of the solution B was increased linearly from 10% to 35% by 55 min. Thereafter, the 35% solution B was passed from 55 min to 65 min. The spray voltage of the ESI probe was 3.5 kV, the capillary temperature was 275°C, and the nitrogen gas was used for the sheath gas, the auxiliary gas, and the collision gas.

The S-Lens RF level was set to 90. In an MS measurement, a selected ion monitoring of m/z 1172.43 was performed in the positive mode. An MS chromatogram acquired was analyzed using the Xcalibur software. The area value of the MS chromatograms was plotted against the amount of added disialyl glycopeptide, thereby absolutely quantifying the amount of the sialyl N-linked glycan A in the breast milk using the standard addition method. This absolute quantitative value and the relative quantitative value of the 153 breast milk samples obtained in "2-3. Measurement of N-Linked glycans" described above were used to calculate the absolute quantitative value of the disialo N-linked glycan A in each milk sample of the 153 samples. For each breast milk sample, the absolute quantitative value of the disialo N-linked glycan B was calculated from a ratio of the area value of the MS chromatogram of the disialo N-linked glycan B to that of the disialo N-linked glycan A.

### (5) Analysis of Relationship Between Various Carbohydrate Components Contents in Breast Milk and Bronchitis

For the 153 breast milk samples, the relationship between the concentrations of N-acetylneuraminic acid, the oligosaccharide, the N-linked glycan, the O-linked glycan, the disialo N-linked glycan A, and the disialo N-linked glycan B and the onset of infant bronchitis was analyzed using the logistic regression. Sex of the infant, a presence or absence of siblings, the method of delivery (natural birth, cesarean section), and a birth weight were used as adjustment factors.

### 2. Results

### (1) Relationship Between Various Carbohydrate Components Contents in Breast Milk and Onset of Infant Bronchitis

For the 153 breast milk samples, the relationship between various carbohydrate components contents (the total N-acetylneuraminic acid, the total oligosaccharides, the total N-linked glycans, and the total O-linked glycans) and the onset of infant bronchitis was analyzed using the logistic regression. As a result, a statistically significant relationship was found between a total N-acetylneuraminic acid concentration and the onset of bronchitis (Table 1). On the other hand, such a relationship was not observed in the total oligosaccharides, the total N-linked glycans, or the total O-linked glycans. An odds ratio and a regression coefficient between the total N-acetylneuraminic acid concentration and the onset of bronchitis were respectively 0.098 and -2.32, indicating that infants who had ever consumed the breast milk with a high concentration of the total N-acetylneuraminic acid had a lower risk of contracting the bronchitis.

**[Table 1]**

| Table 1. Relationship between the onset of bronchitis and the content of various carbohydrate components in the breast milk | | | |
|---|---|---|---|
| Glycan | Odds ratio 95% Cl | Regression coefficient SE | P-value |
| Total oligosaccharide | 0.47 (0.072-3.02) | -0.761 (0.954) | 0.425 |
| Total N-linked glycan | 0.39 (0.10-1.45) | -0.954 (0.678) | 0.159 |
| Total O-linked glycan | 0.76 (0.34-1.68) | -0.279 (0.408) | 0.495 |
| Total N-acetylneuraminic acid | 0.098 (0.012-0.77) | -2.32 (1.05) | 0.027 |

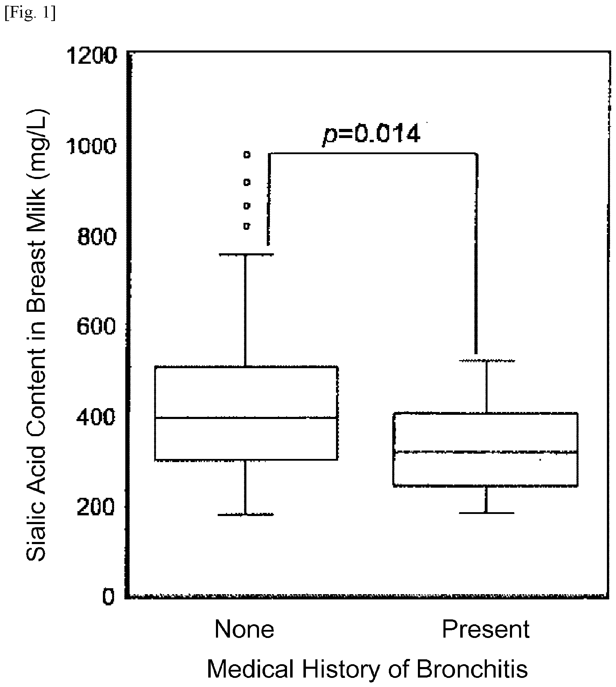

The breast milk samples were divided into two groups, based on whether or not the infants had ever contracted the bronchitis, comparing the total N-acetylneuraminic acids concentrations in the breast milk. As a result, the breast milk consumed by infants who had never contracted the bronchitis contained a significantly higher concentration of the total N-acetylneuraminic acid than the breast milk consumed by the infants who had ever contracted the bronchitis (Fig. 1). This indicates that a nutritional composition for infants containing the same level of N-acetylneuraminic acid as the breast milk consumed by the infants who had never contracted the bronchitis is effective for reducing the risk of contracting the bronchitis. A distribution range of the total N-acetylneuraminic acids concentrations in the breast milk consumed by the infants who had never contracted the bronchitis was 181-977 mg/L, with a median of 395 mg/L (Table 2). The distribution of the total N-acetylneuraminic acids concentrations was, different from a normal distribution, a distribution spreading out its tail towards its higher end of concentrations. Such a distribution is seen in the distribution of heights and weights of the infants, and the 3rd to 97th percentile of the overall distribution is used as standards when creating growth curves for infants (Infant Physical Development Survey Report 2000). Based on this, the 3rd to 97th percentile was used as a standard range of the total N-acetylneuraminic acids concentrations in the breast milk. A minimum value of the total N-acetylneuraminic acid concentration in the breast milk consumed by the infants who had never contracted the bronchitis was 221 mg/L corresponding to the 3rd percentile of the distribution. Total N-acetylneuraminic acids contents in the breast milk was divided into two groups, samples with a content of 246 mg/L or more and samples with a content of 246 mg/L or less, and the relationship with whether or not the infants had contracted the bronchitis was subjected to a Fisher's exact test, to achieve p<0.05 or less. Therefore, the risk of contracting the bronchitis is proved to be reduced when the total N-acetylneuraminic acid content is 246 mg/L or more in the breast milk.

Thus, 221 mg/L or more of N-acetylneuraminic acid is required to be included in a prepared milk as the nutritional composition for infants preventing the onset of bronchitis. Preferably, 246 mg/L or more is desired to be included. Assuming that the prepared milk concentration is 13%, the nutritional composition for infants needs to contain at least 170 mg or more of N-acetylneuraminic acid per 100 g of solid matter. Preferably, 189 mg or more of N-acetylneuraminic acid is desired to be contained.

A mechanism of the onset of bronchitis is the same in infants and adults, so containing this nutritional composition for infants is considered to be effective for adults and elderly people who are at high risk of bronchitis. Thus, this composition is also effective as a nutritional composition for adults containing the same N-acetylneuraminic acid content as above.

**[Table 2]**

| Table 2. Distribution of N-acetylneuraminic acid concentrations in the breast milk of mothers whose infants never had bronchitis | | | | | | | |
|---|---|---|---|---|---|---|---|
| N-acetylneuraminic acid | Percentile (%) | | | | | | |
| | 0 | 3 | 25 | 50 | 75 | 97 | 100 |
| mg/L | 181 | 221 | 303 | 395 | 506 | 756 | 977 |

### (2) Search for N-acetylneuraminic Acid Compound for Preventing Infant Bronchitis

The above results elucidated that infants who consumed the breast milk with a high total N-acetylneuraminic acid content have a lower risk of contracting the bronchitis. Most of N-acetylneuraminic acids in the breast milk exist as glycans of the oligosaccharides or glycoproteins, therefore we explored what molecular form of N-acetylneuraminic acid contributes to the prevention of bronchitis.

For the oligosaccharides (2 types), the N-linked glycans (6 types), and the O-linked glycans (5 types) which are bound to N-acetylneuraminic acids detected in the breast milk, a relevance between each concentration and the onset of bronchitis in infants was analyzed using the logistic regression. Some of the analysis results are shown in Table 3.

As a result of the analysis, the statistically significant relationship between 6'-SL (p=0.035), the disialo N-linked glycan A (p=0.038), and the disialo N-linked glycan B (p=0.046) and the onset of bronchitis. On the other hand, such a relationship was not observed with the 3'-SL content.

The regression coefficients and the odds ratios between 6'-SL and the disialo N-linked glycans A and B and the onset of bronchitis were negative and less than 1, respectively, indicating that the infants who consumed the breast milk with high contents of 6'-SL and the disialo N-linked glycan A and B have the lower risk of contracting the bronchitis.

**[Table 3]**

| Table 3. Relationship between the onset of infant bronchitis and the N-acetylneuraminic acid-containing glycans in the breast milk | | | |
|---|---|---|---|
| Glycan | Odds ratio 95% Cl | Regression coefficient SE | P-value |
| 6'-SL | 0.26 (0.073-0.91) | -1.358 (0.643) | 0.035 |
| 3'-SL | 0.53 (0.14-2.00) | -0.626 (0.672) | 0.351 |
| Disialo N-linked glycan A | 0.15 (0.024-0.90) | -1.918 (0.922) | 0.038 |
| Disialo N-linked glycan B | 0.24 (0.061-0.98) | -1.407 (0.706) | 0.046 |
| α-2,6-disialo N-linked glycan | 0.17 (0.035-0.87) | -1.748 (0.823) | 0.034 |

### (3) Confirmation of Preventive Effect of 6'-SL on Infant Bronchitis

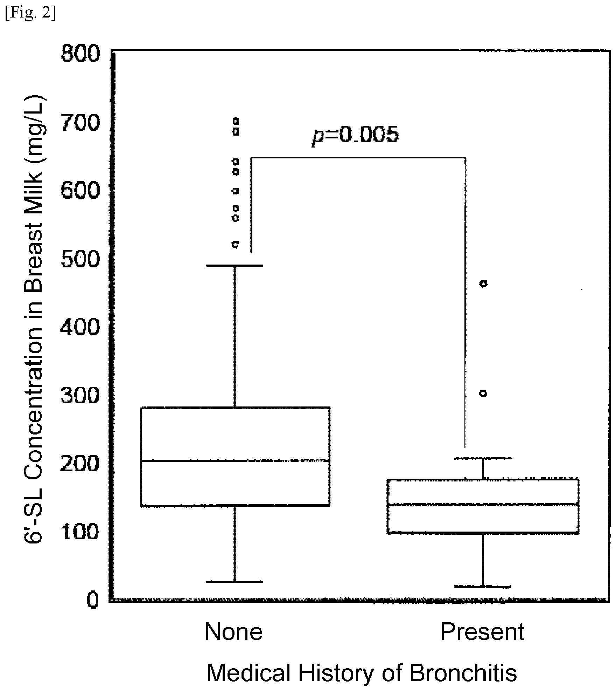

The breast milk samples were divided into two groups, based on whether or not the infants had contracted the bronchitis, comparing 6'-SL concentrations in the breast milk. Consequently, the amount of 6'-SL was significantly higher in the breast milk consumed by the infants who had never contracted the bronchitis than in the breast milk consumed by the infants who had ever contracted the bronchitis (Fig. 2). This indicates that the nutritional composition for infants, which contains the same level of 6'-SL concentration as the breast milk consumed by the infants who had never contracted the bronchitis, are effective for reducing the risk of contracting the bronchitis with a use of the nutritional compositions for infants. The distribution range of 6'-SL concentrations in the breast milk consumed by the infants who had never contracted the bronchitis was 25.7 to 701 mg/L, with the median of 203 mg/L (Table 4).

Similarly to the distribution of N-acetylneuraminic acids, the distribution of 6'-SL concentration in the breast milk is not a normal distribution but a distribution spreading out its tail towards its higher end of concentrations. Therefore, the 3rd to 97th percentile was set as the range of 6'-SL concentrations in the breast milk. 6'-SL concentrations in the breast milk consumed by the infants who had never contracted the bronchitis are considered to be 61.0 mg/L or more corresponding to the 3rd percentile or more. The samples were divided into two groups, i.e. samples with 6'-SL concentrations of 135 mg/L or more and those with the concentrations of 135 mg/L or less in breast milk, and their relationship with the presence or absence of the onset of bronchitis was evaluated using Fisher's exact test. The test results were p<0.05, indicating that 135 mg/L or more of 6'-SL concentration in the breast milk reduces the risk of contracting the bronchitis.

**[Table 4]**

| Table 4. Distribution of N-acetylneuraminic acid concentrations in the breast milk of mothers whose infants never had bronchitis | | | | | | | |
|---|---|---|---|---|---|---|---|
| | Percentile (%) | | | | | | |
| | 0 | 3 | 25 | 50 | 75 | 97 | 100 |
| 6'-SL (mg/L) | 25.7 | 61.0 | 138 | 203 | 281 | 599 | 701 |
| Disialo N-linked glycan A (µmol/L) | 0.0740 | 0.0947 | 0.221 | 0.302 | 0.461 | 1.70 | 2.88 |
| Disialo N-linked glycan B (µmol/L) | 0.0853 | 0.122 | 0.319 | 0.599 | 0.889 | 3.57 | 5.06 |
| α-2,6-disialo N-linked glycan (µmol/L) | 0.179 | 0.257 | 0.557 | 0.933 | 1.36 | 5.30 | 6.76 |

Hence, for the nutritional composition for infants preventing the onset of bronchitis, a milk after preparation needs to contains 61.0 mg/L or more of 6'-SL. Preferably, 135 mg/L or more is desirably contained. Assuming that the prepared milk concentration is 13%, the nutritional composition for infants needs to contain at least 46.9 mg or more of 6'-SL per 100 g of solid matter. Preferably, 104 mg or more of 6'-SL is desired to be contained. The mechanism of the onset of bronchitis is the same in the infants and adults, therefore containing this nutritional composition for infants is also considered to be effective for the adults and elderly people who are at a high risk of bronchitis. Hence, this composition is effective as the nutritional composition for adults with the same 6'-SL content as above.

### (4) Confirmation of Preventive Effect of Disialo N-Linked Glycan A on Infant bronchitis.

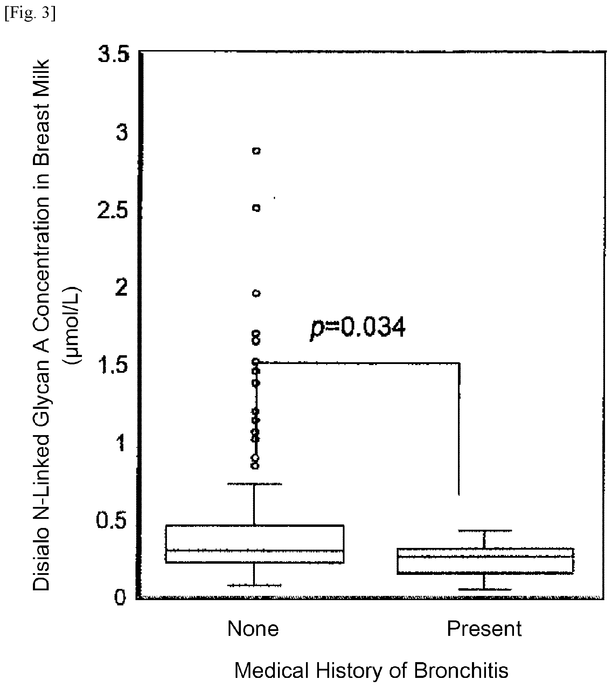

The breast milk samples were divided into two groups, based on whether or not the infants had contracted the bronchitis, comparing the concentrations of the disialo N-linked glycans A in the breast milk. As a result, the amount of the disialo N-linked glycan A was significantly higher in the breast milk consumed by the infants who had never contracted the bronchitis than in the breast milk consumed by the infants who had contracted the bronchitis (Fig. 3). This indicates that the nutritional composition for infants containing the disialo N-linked glycan A concentration at the same level as that of the breast milk consumed by the infants had never contracted the bronchitis is effective for reducing the risk of contracting the bronchitis with the use of the nutritional compositions for infants. The distribution range of the disialo N-linked glycan A concentrations in the breast milk consumed by the infants had never contracted the bronchitis was 0.0740 to 2.88 µmol/L, with the median of 0.302 µmol/L (Table 4). Similarly to the distribution of N-acetylneuraminic acids, that of the disialo N-linked glycan A concentrations in the breast milk was not the normal distribution, but the distribution spreading out its tail towards its higher end of concentrations. Hence, the 3rd to 97th percentiles were set as the range of the disialo N-linked glycan A concentrations in the breast milk. The disialo N-linked glycan A concentrations in the breast milk consumed by the infants who had never contracted the bronchitis are considered to be 0.0947 µmol/L or more, which corresponds to the 3rd percentile or more. The breast milk samples were divided into two groups, which are the samples with the disialo N-linked glycan A concentration of 0.313 µmol/L or more in the breast milk, and the samples with the disialo N-linked glycan A concentration of 0.313 µmol/L or less, evaluating the relationship with the presence or absence of the onset of bronchitis using the Fisher's exact test. The test results were p<0.05, indicating that 0.313 µmol/L or more of the disialo N-linked glycan A concentration in the breast milk reduces the risk of contracting the bronchitis.

Hence, for the nutritional composition for infants preventing the onset of bronchitis, milk after preparation needs to contain 0.0947 µmol/L or more of the disialo N-linked glycan A. Preferably, 0.313 µmol/L or more is desired to be contained. Assuming that the prepared milk concentration is 13%, the nutritional composition for infants needs to contain a minimum of 0.0728 µmol or more of the disialo N-linked glycan A per 100 g of solid matter. Preferably, 0.241 µmol or more of the disialo N-linked glycan A is desired to be contained. Since the mechanism of the onset of bronchitis is the same in the infants and adults, containing this nutritional composition for infants is also considered to be effective for the adult and elderly people who are at high risk of the bronchitis. Therefore, this composition is also effective as the nutritional composition for adults containing the same content of the disialo N-linked glycan A as above.

### (5) Confirmation of Preventive Effect of Disialo N-Linked Glycan B on Infant Bronchitis

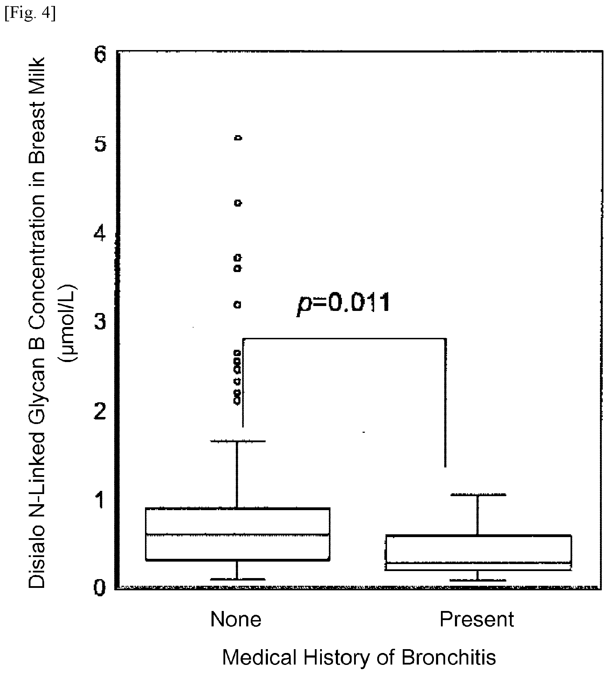

The breast milk samples were divided into two groups, based on whether or not the infants had ever contracted the bronchitis, comparing the disialo N-linked glycan B concentrations in the breast milk. Consequently, the amount of the disialo N-linked glycan B was significantly higher in the breast milk consumed by the infants who had never contracted the bronchitis than in the breast milk consumed by the infants who had ever contracted the bronchitis (Fig. 4). This indicates that the nutritional composition for infants containing the disialo N-linked glycan B concentration at the same level as the breast milk consumed by the infants who had never contracted the bronchitis is effective for reducing the risk of contracting the bronchitis with use of the nutritional compositions for infants. The range of the disialo N-linked glycan B concentrations in the breast milk consumed by the infants who had never contracted the bronchitis was 0.0853 to 5.06 µmol/L, with the median of 0.599 µmol/L (Table 4). Similarly to the distribution of N-acetylneuraminic acids, the distribution of the disialo N-linked glycan B concentrations in the breast milk was not the normal distribution, but the distribution spreading out its tail towards its higher end of concentrations. Hence, the 3rd to 97th percentiles were set as the range of the disialo N-linked glycan B concentrations in the breast milk. The disialo N-linked glycan B concentrations in the breast milk consumed by the infants who had never contracted the bronchitis are considered to be 0.122 µmol/L or more which corresponds to the 3rd percentile or more. The samples were divided into two groups, i.e., samples with 0.245 µmol/L or more and those with 0.245 µmol/L or less of disialo N-linked glycan B concentrations in breast milk, and their relationship with the presence or absence of the onset of bronchitis was evaluated using Fisher's exact test. The test results were p<0.05 or less, indicating that 0.245 µmol/L or more of the disialo N-linked glycan B concentrations in the breast milk lowers the risk of contracting the bronchitis.

Therefore, for the nutritional composition for infants preventing the onset of bronchitis, milk after preparation needs to contain 0.122 µmol/L or more of the disialo N-linked glycan B. Preferably, 0.245 µmol/L or more is desired to be contained. Assuming that the prepared milk concentration is 13%, the nutritional composition for infants needs to contain a minimum of 0.0935 µmol or more of the disialo N-linked glycan B per 100 g of solid matter. Preferably, 0.188 µmol or more of the disialo N-linked glycan B is desired to be contained. Since the mechanism of contracting the bronchitis is the same in infants and adults, containing this infant nutritional composition is also effective for the adults and elderly people who are at high risk of bronchitis. Hence, this composition is also effective as an adult nutritional composition with the same disialo N-linked glycan B content as above.

### (6) Determination of Structures of Disialo N-linked Glycans A and B

For investigating structures of the disialo N-linked glycans A and B in the breast milk which were proved to be related to the onset of infant bronchitis, a retention time of the LC/MS chromatogram was compared with that of an α-2,3 or α-2,6-disialo N-linked glycan standard preparation. As a result, the retention times of the disialo N-linked glycans A and B were both consistent with those of α-2,6-disialo N-linked glycan standard preparation.

In a case that the disialo N-linked glycans A and B were treated with a nonspecific sialidase, degradation of the glycans was observed. On the other hand, in a case that they were treated with a sialidase which specifically cleaves the α-2,3-linked N-acetylneuraminic acids, the glycans were not degraded. From the above, the disialo N-linked glycans A and B are demonstrated to be α-2,6-disialo N-linked glycans represented by the following structure.

### <Disialo N-Linked Glycan A>

Neu5Acα2-6Galβ1-4GlcNAcβ1-2Manα1-3 (Neu5Acα2-6Galβ1-4GlcNAcβ1-2Manα1-6) Manβ1-4GlcNAcβ1-4GlcNAc

### <Disialo N-Linked Glycan B>

Neu5Acα2-6Galβ1-4GlcNAcβ1-2Manα1-3 (Neu5Acα2-6Galβ1-4GlcNAcβ1-2Manα1-6) Manβ1-4GlcNAcβ1-4 (Fucα1-6) GlcNAc

### (7) Confirmation of Preventive Effect of α-2,6 Disialo N-Linked Glycans on Infant Bronchitis

The glycan structures of the disialo N-linked glycans A and B have an only difference whether or not a fucose forms a single molecule bound. Both of them have in common that two molecules of N-acetylneuraminic acid are α-2,6-bound to galactose at a non-reducing terminal. Therefore, N-linked glycans with this α-2,6-disialo bond was considered to have a possibility of being relevant to the infant bronchitis. Hence, the relationship between the amounts of substances (molar concentrations) of α-2,6-disialo N-linked glycans in the breast milk, which are the sums of the amounts of substances (molar concentrations) of the disialo N-linked glycans A and B in the breast milk, and the bronchitis was analyzed by the logistic regression. The results were p=0.034, revealing the statistically significant relationship between the amounts (molar concentrations) of α-2,6-disialo N-linked glycans in the breast milk and the onset of bronchitis (Table 3). This relevance has a negative regression coefficient and an odds ratio of less than 1, indicating that infants who consumed the breast milk with high amounts (molar concentrations) of α-2,6-disialo N-linked glycans in the breast milk had a lower risk of contracting the bronchitis.

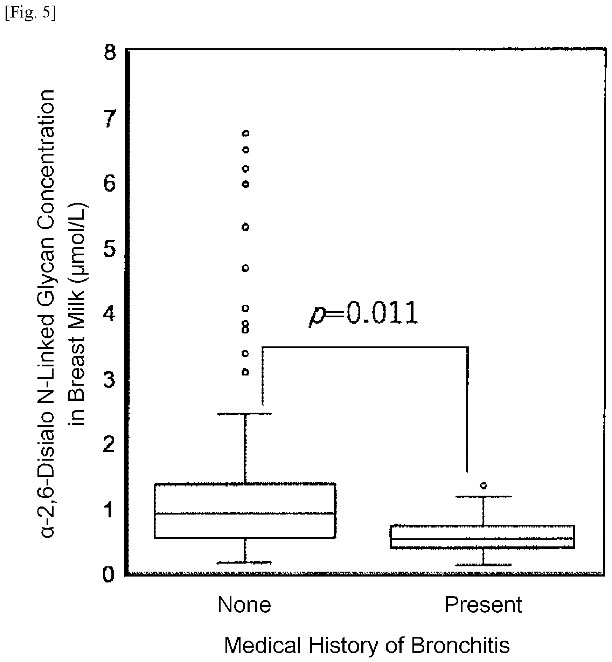

The breast milk samples were divided into two groups, based on whether or not the infants had ever contracted the bronchitis, comparing the amounts (molar concentrations) of α-2,6-disialo N-linked glycans in the breast milk. As a result, the amounts (molar concentrations) of α-2,6-disialo N-linked glycans in the breast milk consumed by the infants who had never contracted the bronchitis were significantly higher than that consumed by the infants who had contracted the bronchitis (Fig. 5). This indicates that the nutritional composition for infants containing α-2,6-disialo N-linked glycans at the same level as the breast milk consumed by the infants who had never contracted the bronchitis is effective for reducing the risk of contracting the bronchitis with use of the nutritional components. The distribution range of the amounts of α-2,6-disialo N-linked glycans in the breast milk consumed by the infants had never contracted the bronchitis was 0.179 to 6.76 µmol/L, with the median of 0.933 µmol/L (Table 4). Similarly to the distribution of N-acetylneuraminic acids, the distribution of α-2,6-disialo N-linked glycans in the breast milk was not a normal distribution, but was the distribution spreading out its tail toward higher end of concentrations. Hence, the concentration range of α-2,6-disialo N-linked glycans in the breast milk was set to the 3rd to 97th percentiles. α-2,6-disialo N-linked glycans in the breast milk consumed by the infants who had never contracted the bronchitis are considered to be 0.257 µmol/L or more, which corresponds to the 3rd percentile or more. The samples were divided into two groups, i.e. samples with 0.440 µmol/L or more and those with 0.440 µmol/L or less of α-2,6-disialo N-linked glycans in the breast milk, and their relationship with the presence or absence of the onset of bronchitis was evaluated using Fisher's exact test. The results were p<0.05, indicating that 0.440 µmol/L or more of α-2,6-disialo N-linked glycans in the breast milk reduces the risk of the onset of bronchitis.

Hence, for the nutritional composition for infants preventing the onset of bronchitis, milk after preparation is required to contain 0.257 µmol/L or more of α-2,6-disialo N-linked glycans. Preferably, 0.440 µmol/L or more is desired to be contained. Assuming that the prepared milk concentration is 13%, the nutritional composition for infants is required to contain at least 0.198 µmol or more of α-2,6-disialo N-linked glycans per 100 g of solid matter. Preferably, 0.338 µmol or more of α-2,6-disialo N-linked glycans were required to be contained. Since the mechanism of the bronchitis is the same in infants and adults, this nutritional composition for infants is also considered to be effective for the adults and elderly people who are at high risk of bronchitis. Therefore, this composition is also effective as the nutritional composition for adults containing the same α-2,6-disialo N-linked glycans as above.

### [Example 1] Preparation of Modified Milk Powder Containing N-Acetylneuraminic acid

Fifty-two point seven (52.7) kg of a whey powder, 239 kg of a skim milk, and 28 g of N-acetylneuraminic acid were dissolved in 500 kg of a water to be mixed with 23.9 kg of a vegetable oil, and then the solution was homogenized. The resulting solution was sterilized, concentrated in a standard method, and dried to obtain 99 kg of milk powder. The milk powder was mixed with 1 kg of vitamins and mineral components to finally obtain 100 kg of milk powder. The amount of N-acetylneuraminic acids in the resulting milk powder was 221 mg / 100 g solid.

### [Example 2] Preparation of Modified Milk Powder Containing 6'-Sialyllactose

Fifty-two point seven (52.7) kg of the whey powder, 239 kg of the skim milk, and 50 g of 6'-sialyllactose were dissolved in 500 kg of the water to be mixed with 23.9 kg of the vegetable oil, and then the solution was homogenized. The resulting solution was sterilized, concentrated in a standard method, and dried to obtain 99 kg of milk powder. The milk powder was mixed with 1 kg of the vitamins and mineral components to finally obtain 100 kg of milk powder. The amount of 6'-sialyllactose in the resulting milk powder was 50 mg/100 g solid.

### [Example 3] Preparation of Milk Powder Containing Glycopeptides

Fifty-two point seven (52.7) kg of the whey powder, 239 kg of the skim milk, and 567 mg of a sialyl glycopeptide were dissolved in 500 kg of the water, and this solution was mixed with 23.9 kg of the vegetable oil to be homogenized. The resulting solution was sterilized, concentrated in a standard method, and dried to obtain 99 kg of milk powder. The milk powder was mixed with 1 kg of the vitamins and mineral components to obtain 100 kg of a prepared milk powder. The amount of α-2,6-disialo N-linked glycans in the resulting milk powder was 0.198 µmol/100 g solid.

### [Example 4] Preparation of Milk Powder Containing Glycopeptide

Fifty-two point seven (52.7) kg of the whey powder, 239 kg of the skim milk, and 421 g of an egg yolk powder were dissolved in 500 kg of the water, and this solution was mixed with 23.9 kg of the vegetable oil to be homogenized. The resulting solution was sterilized, concentrated in a standard method, and dried to obtain 99 kg of the powdered milk. The powdered milk was mixed with 1 kg of the vitamins and mineral components to finally obtain 100 kg of the prepared powdered milk. α-2,6-disialo N-linked glycan of the resulting powdered milk was 0.198 µmol/100 g solid.

### [Example 5] Manufacturing of Supplements

One (1) g of N-acetylneuraminic acid powder was mixed with 40 g of an equal mixture of vitamins C and citric acids, 100 g of a granulated sugar, and 60 g of an equal mixture of a corn starch and a lactose. The mixture was packed into a stick-shaped bag, manufacturing supplements for preventing bronchitis of the present invention.

### [Example 6] Manufacturing of Supplements

Ten (10) mg of α-2,6-disialo N-linked glycan was mixed with 40 g of the equal mixture of vitamin C and citric acid, 100 g of the granulated sugar, and 60 g of the equal mixture of cornstarch and lactose. The mixture was packed into the stick-shaped bag to manufacturing the supplements for preventing bronchitis of the present invention.

### [Example 7] Manufacturing of Beverages

Ingredients were mixed according to a composition shown in Table 5, and then they were filled into a container and were heat-sterilized to manufacture the beverages for preventing bronchitis of the present invention.

### [Table 5]

**Table 5.**

| | Content (weight %) |
|---|---|
| N-acetylneuraminic acid | 2. 5 |
| Sugar | 7. 5 |
| Citric acid | 0. 6 |
| Apple juice | 10. 0 |
| Water | 79. 4 |

### [Example 8] Manufacturing of Beverages

The ingredients were mixed according to the composition shown in Table 6, and they were filled into the container and then were heat-sterilized to manufacture the beverages for preventing bronchitis of the invention.

### [Table 6]

**Table 6.**

| | Content (weight %) |
|---|---|
| α-2,6-disialo N-linked glycan | 0. 1 |
| Sugar | 7. 5 |
| Citric acid | 0. 6 |
| Apple juice | 10. 0 |
| Water | 81. 8 |

### INDUSTRIAL APPLICABILITY

According to the present invention, a nutritional composition containing N-acetylneuraminic acid as an active ingredient can prevent the onset of bronchitis. The component is effective not only for infants but also for elderly people and people having underlying diseases who are at high risk of bronchitis. Therefore, the nutritional composition of the invention can also prevent the onset of bronchitis in adults and the elderly.

## Claims

1. A nutritional composition for preventing bronchitis comprising N-acetylneuraminic acid as an active ingredient.

2. The nutritional composition according to claim 1, wherein N-acetylneuraminic acid is bound to any one or more of a protein, a peptide, an oligosaccharide, or a lipid.

3. The nutritional composition according to claim 1 or 2, wherein N-acetylneuraminic acid is an N-acetylneuraminic acid.

4. The nutritional composition according to claim 3, wherein N-acetylneuraminic acid exists as 6'-sialyllactose.

5. The nutritional composition according to claim 3, wherein N-acetylneuraminic acid exists as an N-linked glycan.

6. The nutritional composition according to claim 5, wherein the N-linked glycan is α-2,6-disialo N-linked glycan.

7. The nutritional composition according to claim 6, wherein α-2,6-disialo N-linked glycan has a structure shown in (1) or (2) below.
(1) Neu5Acα2-6Galβ1-4GlcNAcβ1-2Manα1-3(Neu5Acα2-6Galβ1-4GlcNAcβ1-2Manα1-6)Manβ1-4GlcNAcβ1-4GlcNAc
(2) Neu5Acα2-6Galβ1-4GlcNAcβ1-2Manα1-3(Neu5Acα2-6Galβ1-4GlcNAcβ1-2Manα1-6)Manβ1-4GlcNAcβ1-4(Fucα1-6)GlcNAc

8. The nutritional composition according to any one of claims 1 to 7, wherein said nutritional composition is for infants.

9. A food and drink for preventing bronchitis comprising the nutritional composition for preventing bronchitis according to any one of claims 1 to 8.

10. A pharmaceutical for preventing bronchitis comprising the nutritional composition for preventing bronchitis according to any one of claims 1 to 8.
